# EUROPEAN PATENT SPECIFICATION

(11) **EP 3 773 841 B1**
(45) Date of publication and mention of the grant of the patent: **07.05.2025**
(21) Application number: 19713440.6
(22) Date of filing: 22.03.2019
(51) Int. Cl.: A61M 16/06, A61M 16/08

(54) **ADJUSTABLE FRAME FOR AN INTERFACE DEVICE**
EINSTELLBARER RAHMEN FÜR EINE SCHNITTSTELLENVORRICHTUNG
CADRE RÉGLABLE POUR UN DISPOSITIF D'INTERFACE

(30) Priority: 30.03.2018 US 201862650333 P
(43) Date of publication of application: 17.02.2021
(73) Proprietor: Koninklijke Philips N.V., 5656 AG Eindhoven (NL)
(72) Inventor: ROTHERMEL, Justin, Edward, 5656 AE Eindhoven (NL); CHODKOWSKI, Lauren, Patricia, 5656 AE Eindhoven (NL); HAIBACH, Richard, Thomas, 5656 AE Eindhoven (NL); STEED, Daniel, 5656 AE Eindhoven (NL); GRASHOW, Jonathan, Sayer, 5656 AE Eindhoven (NL)
(74) Representative: Philips Intellectual Property & Standards
(86) International application number: PCT/EP2019/057231
(87) International publication number: WO 2019/185474

(56) References cited:
- WO-A1-2016/193859
- WO-A1-2017/124152
- US-A1- 2010 018 534
- US-A1- 2015 182 719
- US-A1- 2015 283 349
- US-A1- 2016 082 217
- US-A1- 2017 266 404
- US-B2- 8 887 727

## Description

### BACKGROUND OF THE INVENTION

### 1. Field of the Invention

The present invention pertains to interface devices for use in delivering a flow of treatment gas to the airway of a user and, more particularly, to adjustable frames for use in such interface devices.

### 2. Description of the Related Art

Many individuals suffer from disordered breathing during sleep. Sleep apnea is a common example of such sleep disordered breathing suffered by millions of people throughout the world. One type of sleep apnea is obstructive sleep apnea (OSA), which is a condition in which sleep is repeatedly interrupted by an inability to breathe due to an obstruction of the airway; typically the upper airway or pharyngeal area. Obstruction of the airway is generally believed to be due, at least in part, to a general relaxation of the muscles which stabilize the upper airway segment, thereby allowing the tissues to collapse the airway. Another type of sleep apnea syndrome is a central apnea, which is a cessation of respiration due to the absence of respiratory signals from the brain's respiratory center. An apnea condition, whether obstructive, central, or mixed, which is a combination of obstructive and central, is defined as the complete or near cessation of breathing, for example a 90% or greater reduction in peak respiratory air-flow.

Those afflicted with sleep apnea experience sleep fragmentation and complete or nearly complete cessation of ventilation intermittently during sleep with potentially severe degrees of oxyhemoglobin desaturation. These symptoms may be translated clinically into extreme daytime sleepiness, cardiac arrhythmias, pulmonary-artery hypertension, congestive heart failure and/or cognitive dysfunction. Other consequences of sleep apnea include right ventricular dysfunction, carbon dioxide retention during wakefulness, as well as during sleep, and continuous reduced arterial oxygen tension. Sleep apnea sufferers may be at risk for excessive mortality from these factors as well as by an elevated risk for accidents while driving and/or operating potentially dangerous equipment.

Even if a patient does not suffer from a complete or nearly complete obstruction of the airway, it is also known that adverse effects, such as arousals from sleep, can occur where there is only a partial obstruction of the airway. Partial obstruction of the airway typically results in shallow breathing referred to as a hypopnea. A hypopnea is typically defined as a 50% or greater reduction in the peak respiratory air-flow. Other types of sleep disordered breathing include, without limitation, upper airway resistance syndrome (UARS) and vibration of the airway, such as vibration of the pharyngeal wall, commonly referred to as snoring.

It is well known to treat sleep disordered breathing by applying a continuous positive air pressure (CPAP) to the patient's airway. This positive pressure effectively "splints" the airway, thereby maintaining an open passage to the lungs. It is also known to provide a positive pressure therapy in which the pressure of gas delivered to the patient varies with the patient's breathing cycle, or varies with the patient's breathing effort, to increase the comfort to the patient. This pressure support technique is referred to as bi-level pressure support, in which the inspiratory positive airway pressure (IPAP) delivered to the patient is higher than the expiratory positive airway pressure (EPAP). It is further known to provide a positive pressure therapy in which the pressure is automatically adjusted based on the detected conditions of the patient, such as whether the patient is experiencing an apnea and/or hypopnea. This pressure support technique is referred to as an auto-titration type of pressure support, because the pressure support device seeks to provide a pressure to the patient that is only as high as necessary to treat the disordered breathing.

Pressure support therapies as just described involve the placement of a patient interface device including a mask component having a soft, flexible sealing cushion on the face of the patient. The mask component may be, without limitation, a nasal mask that covers the patient's nose, a nasal/oral mask that covers the patient's nose and mouth, or a full face mask that covers the patient's face. Such patient interface devices may also employ other patient contacting components, such as forehead supports, cheek pads and chin pads. The patient interface device is typically secured to the patient's head by a headgear component. The patient interface device is connected to a gas delivery tube or conduit and interfaces the pressure support device with the airway of the patient, so that a flow of breathing gas can be delivered from the pressure/flow generating device to the airway of the patient.

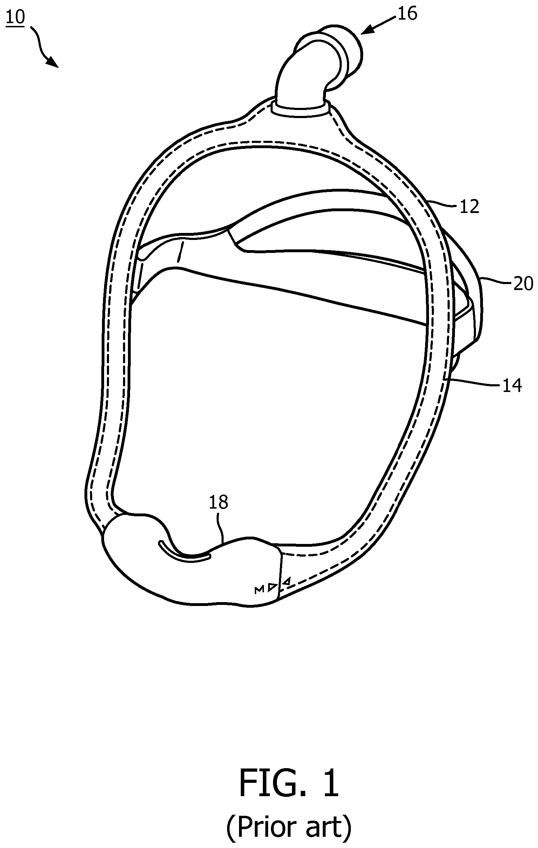

CPAP masks with air delivery tubing integrated into headgear and frames is becoming more commonplace and is seen, for example, without limitation, in masks such as the DreamWear mask manufactured and sold by Philips Respironics, an example of which is shown generally at 10 in FIG. 1. DreamWear mask 10 includes a frame 12 having an integrated gas pathway 14 (shown in hidden line) that carries the therapy air from an inlet 16 which is structured to be positioned at the top of a user's head (not shown) to a nasal interface 18 which is structured to sealing engage about the nares of the user. Mask 10 further includes an adjustable headgear 20 for assisting in securing frame 12 to the head of a user.

While headgear 20 provides some adjustability for sizing, for most sizing the total overall perimeter of frame 12 is changed to affect patient fit and is done so by replacing the entire frame 12 with a different size frame. However, as frame 12 is quite a large part on mask 10, there are three notable disadvantages to such arrangement: i) DME's (Durable Medical Equipment) find such arrangement economically burdensome due to space and cost to stock; ii) RT's (Respiratory Therapists) find the ease of use to change the size (disassemble headgear, elbow, mask and switch the frame) cumbersome; and iii) manufacturing of such large continuous pieces is difficult and generally requires a high number of injection molding presses.

The first two of these issues may result in patients not receiving the proper size frame 12. Having a simpler way to adjust the frame allows one to improve ease of use, reduce the amount of space needed to store a sufficient quantity of the product, and would reduce the cost burden. Resolving such problems with masks of this type could greatly increase the use of the product by the DME, RT and consequently the patient.

WO 2017/124152 discloses an inflatable positioning and stabilizing structure for a patient interface.

US 2010/0018534 discloses a respiratory mask for continuous positive airway pressure treatment including a cushion adapted to be positioned against the face of a patient.

### SUMMARY OF THE INVENTION

The invention is defined by the claims.

Accordingly, it is an object of the present invention to provide a system that overcomes the shortcomings of conventional interface devices used in delivering a flow of a treatment gas to the airway of a patient.

As one aspect of the invention, a frame for use in an interface device having a sealing assembly for delivering a flow of treatment gas to the airway of a patient is provided according to claim 1.

The first end of the first frame member and the first end of the second frame member may be merged together in a single inlet conduit which is structured to be coupled to the conduit supplying the flow of treatment gas.

As another aspect of the present invention, an interface device for use in delivering a flow of treatment gas to the airway of a user of the device is provided according to claim 3.

The first end of the first frame member and the first end of the second frame member may be merged together in a single inlet conduit which is structured to be coupled to the conduit supplying the flow of treatment gas.

As another aspect of the present invention, a system for use in delivering a flow of a breathing gas to the airway of a patient is provided according to claim 5.

These and other objects, features, and characteristics of the present invention, as well as the methods of operation and functions of the related elements of structure and the combination of parts and economies of manufacture, will become more apparent upon consideration of the following description and the appended claims with reference to the accompanying drawings, all of which form a part of this specification, wherein like reference numerals designate corresponding parts in the various figures. It is to be expressly understood, however, that the drawings are for the purpose of illustration and description only and are not intended as a definition of the limits of the invention as defined by the claims.

### BRIEF DESCRIPTION OF THE DRAWINGS

FIG. 1 is an isometric view of a conventional patient interface device for use in delivering a flow of treatment gas upon which the disclosed concept improves;
FIG. 2 is a partially schematic view of an airway pressure support system including a patient interface device in accordance with one example embodiment of the present invention shown disposed on the head of a user;
FIG. 3 is a top view of the patient interface device of FIG. 2 shown disposed on the head of a user;
FIG. 4 is an isometric view of a patient interface device which includes a removable hub member, not according to the present invention;
FIG. 5 is an example of three different sized central hub members for use in the patient interface device of FIG. 4;
FIGS. 6A and 6B are examples of central hub members that may be employed in the patient interface device of FIG. 4;
FIG. 7A is a front elevation view of selected portions of a frame of a patient interface device, not according to the present invention, shown disposed in a first positioning;
FIG. 7B is a front elevation view of the selected portions of the frame of FIG. 7A shown in a second positioning, different from the first positioning;
FIG. 7C is an isometric view of the central hub member of the frame of FIGS. 7A and 7B;
FIG. 7D is an isometric view of a stiffener in accordance with an example embodiment of the present invention;
FIG. 7E is an isometric view of a hollow arm member for use in the frame of FIGS. 7A and 7B including a rigid clip in accordance with an example embodiment of the present invention;
FIG. 7F is an isometric view of the rigid clip of FIG. 7E;
FIG. 7G is a simplified sectional view showing the interaction between a locking tab of a reduced portion of a central member and the rigid clip and a portion of the arm member of FIG. 7E;
FIG. 8 is an isometric view of an example patient interface device, not according to the present invention, for use in delivering a flow of treatment gas;
FIG. 9A is a partially exploded isometric view of a portion of the patient interface device of FIG. 8;
FIG. 9B is an assembled view of the portion of FIG. 9A;
FIG. 9C is exploded and non-exploded sectional views taken along line C-C of FIG. 9B;
FIG. 10A is a partially exploded isometric view of a portion of the patient interface device of FIG. 8;
FIG. 10B is an assembled view of the portion of FIG. 10A;
FIG. 10C is a sectional view taken along line C-C of FIG. 10B;
FIG. 11 is an isometric view of another example patient interface device, not according to the present invention, for use in delivering a flow of treatment gas;
FIG. 12A is an example hub member for use in the patient interface device of FIG. 11;
FIG. 12B shows the example hub member of FIG. 12A disposed with a portion of the frame of the patient interface device of FIG. 11 in a first positioning;
FIG. 12C shows the example hub member of FIG. 12A disposed with a portion of the frame of the patient interface device of FIG. 11 in a second positioning;
FIG. 13A is an example hub member for use in the patient interface device of FIG. 11;
FIG. 13B shows the example hub member of FIG. 13A disposed with a portion of the frame of the patient interface device of FIG. 11 in a first positioning;
FIG. 13C shows the example hub member of FIG. 13A disposed with a portion of the frame of the patient interface device of FIG. 11 in a second positioning;
FIG. 14A is an example hub member for use in the patient interface device of FIG. 11;
FIG. 14B shows the example hub member of FIG. 14A disposed with a portion of the frame of the patient interface device of FIG. 11 in a first positioning;
FIG. 14C shows the example hub member of FIG. 14A disposed with a portion of the frame of the patient interface device of FIG. 11 in a second positioning; and
FIG. 14D shows the example hub member of FIG. 14A disposed with a portion of the frame of the patient interface device of FIG. 11 in a third positioning.

### DETAILED DESCRIPTION OF EXEMPLARY EMBODIMENTS

As required, detailed embodiments of the present invention are disclosed herein; however, it is to be understood that the disclosed embodiments are merely exemplary of the invention, which may be embodied in various forms. Therefore, specific structural and functional details disclosed herein are not to be interpreted as limiting, but merely as a basis for the claims and as a representative basis for teaching one skilled in the art to variously employ the present invention in virtually any appropriately detailed structure.

As used herein, the singular form of "a", "an", and "the" include plural references unless the context clearly dictates otherwise. As used herein, the statement that two or more parts or components are "coupled" shall mean that the parts are joined or operate together either directly or indirectly, i.e., through one or more intermediate parts or components, so long as a link occurs. As used herein, "directly coupled" means that two elements are directly in contact with each other. As used herein, "fixedly coupled" or "fixed" means that two components are coupled so as to move as one while maintaining a constant orientation relative to each other. As used herein, "selectively coupled" means that two components are coupled in a manner which allows for the components to be readily coupled or uncoupled in a predictable, repeatable manner without damaging either of the components. Unless particularly described otherwise herein, any components which are described merely as being "coupled", may also be "fixedly" or "selectively" coupled without varying from the scope of the present invention.

As used herein, the word "unitary" means a component is created as a single piece or unit. That is, a component that includes pieces that are created separately and then coupled together as a unit is not a "unitary" component or body. As used herein, the statement that two or more parts or components "engage" one another shall mean that the parts exert a force against one another either directly or through one or more intermediate parts or components. As used herein, the term "number" shall mean one or an integer greater than one (i.e., a plurality).

Directional phrases used herein, such as, for example and without limitation, top, bottom, left, right, upper, lower, front, back, and derivatives thereof, relate to the orientation of the elements shown in the drawings and are not limiting upon the claims unless expressly recited therein.

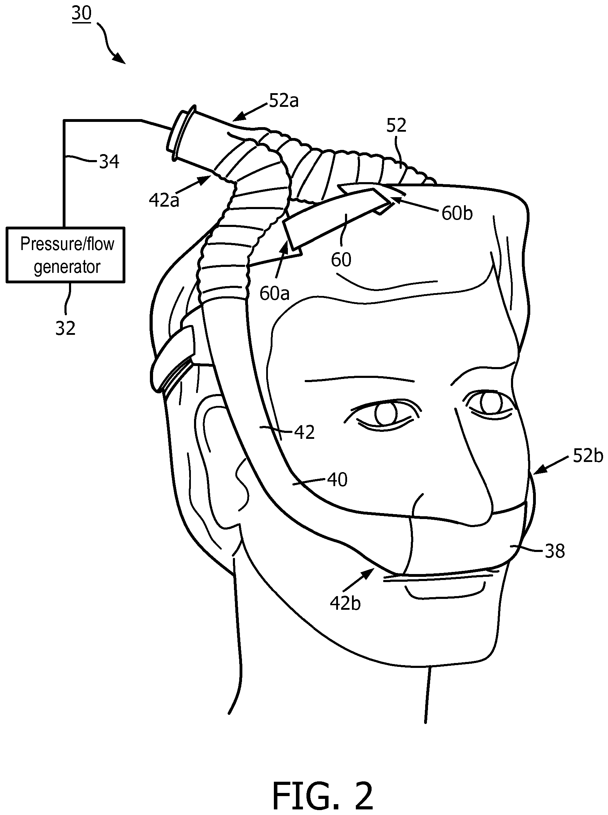

An example airway pressure support system 30 according to one particular, non-limiting exemplary embodiment of the present invention is shown in FIG. 2. System 30 includes a pressure/flow generator 32, a delivery conduit 34, and patient interface device 36 disposed on the head (not numbered) of a patient. Pressure/flow generator 32 is structured to generate a flow of breathing gas which may be heated and/or humidified. Pressure/flow generator 32 may include, without limitation, ventilators, constant pressure support devices (such as a continuous positive airway pressure device, or CPAP device), variable pressure devices (e.g., BiPAP^{®}, Bi-Flex^{®}, or C-Flex^{™} devices manufactured and distributed by Philips Respironics of Murrysville, Pennsylvania), and auto-titration pressure support devices. Delivery conduit 34 is structured to communicate the flow of breathing gas from pressure/flow generator 32 to patient interface device 36. Delivery conduit 34 and patient interface device 36 are often collectively referred to as a patient circuit.

A BiPAP^{®} device is a bi-level device in which the pressure provided to the patient varies with the patient's respiratory cycle, so that a higher pressure is delivered during inspiration than during expiration. An auto-titration pressure support system is a system in which the pressure varies with the condition of the patient, such as whether the patient is snoring or experiencing an apnea or hypopnea. For present purposes, pressure/flow generating device 32 is also referred to as either pressure generating device or gas flow generating device, because flow results when a pressure gradient is generated. The present invention contemplates that pressure/flow generating device 32 is any conventional system for delivering a flow of gas to an airway of a patient or for elevating a pressure of gas at an airway of the patient, including the pressure support systems summarized above and non-invasive ventilation systems. Although described herein in example embodiments wherein a pressurized flow of gas is utilized, it is to be appreciated that embodiments of the invention as described herein could also be readily employed in other generally non-pressurized applications (e.g., without limitation, in high flow therapy applications).

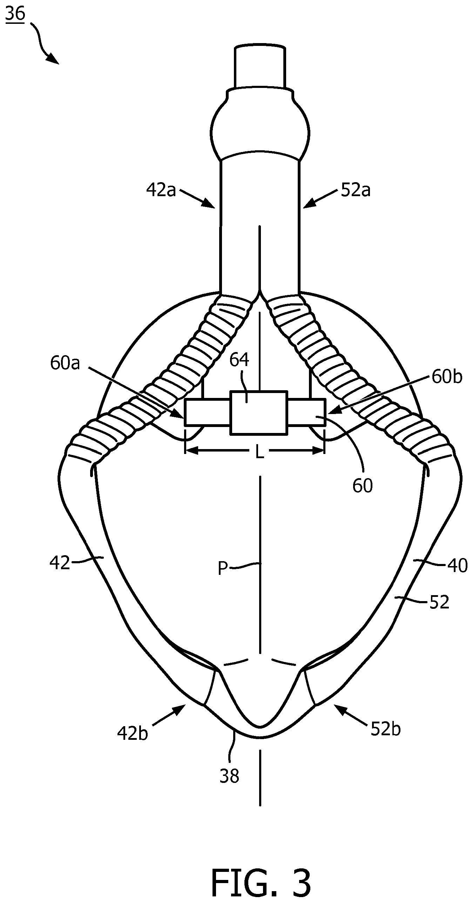

In the exemplary embodiment of FIG. 2, patient interface device 36 includes a patient sealing assembly 38 coupled to an example adjustable frame 40 according to one example embodiment of the present invention. In the example embodiment illustrated in FIGS. 2 and 3, patient sealing assembly 38 is a nasal pillow, however, it is to be appreciated that other types of patient sealing assemblies, such as, without limitation, a nasal/oral mask, a nasal cushion, or any other suitable arrangements which facilitate the delivery of the flow of breathing gas to the airway of a patient may be substituted for patient sealing assembly 38 while remaining within the scope of the present invention.

Continuing to refer to FIGS. 2 and 3, frame 40 includes a first frame member 42 having a first end 42a which is structured to be coupled to a conduit (e.g., delivery conduit 34) supplying the flow of treatment gas and an opposite second end 42b coupled to sealing assembly 38. First frame member 42 includes a flow passage (not numbered) defined therein which extends between first end 42a and second end 42b which is structured to convey a flow of treatment gas (such as provided by pressure/flow generator 32) through first frame member 42. Frame 40 further includes a second frame member 52 of similar arrangement as first frame member 42. Accordingly, second frame member 52 has a first end 52a structured to be coupled to a conduit supplying the flow of treatment gas and an opposite second end 52b coupled to sealing assembly 38. Second frame member 52 also includes a passage (not numbered) defined therein which extends between first end 52a second end 52b which is structured to convey a flow of treatment gas (such as provided by pressure/flow generator 32) through second frame member 52. First frame member 42 and second frame member 52 are coupled together at or near first ends 42a and 52a thereof via any suitable coupling mechanism. In the example embodiment of the present concept illustrated in FIGS. 2 and 3, first end 42a of first frame member 42 and first end 52a of second frame member 52 are merged together in a single inlet conduit 56 which is structured to be coupled to a conduit, such as conduit 34, supplying a flow of treatment gas.

Frame 40 is constructed such that when positioned on the head of a patient (e.g., such as shown in FIGS. 2 and 3) first frame member 42 and second frame member 52 are biased toward each other. For example, as shown in FIG. 3, when frame 40 is disposed on the head of a patient, first frame member 42 is disposed on a first side of, and biased generally toward, a reference plane P which generally bisects the face and head of the patient and passes through sealing assembly 38 and second frame member 52 is disposed on a second side of, and biased toward, reference plane P opposite the first side.

In one example embodiment, such biasing is accomplished by forming first and second frame members 42 and 52 from a flexibly resilient material in a first positioning narrower than the width of a human head. Hence, when flexed to a wider second positioning, such as when frame 40 is positioned on a human head, first and second frame members 42 and 52 are distorted from such first positioning and thus are biased toward each other due to the inherent construction of members 42 and 52.

According to the invention, frame further includes a strap 60 spanning between a first end 60a and an opposite second end 60b. First end 60a is coupled to first frame member 42 at a location between first end 42a and second end 42b of first frame member 42 while second end 60b is coupled to second frame member 52 at a location between first end 52a and second end 52b such that strap 60 spans between first frame member 42 and second frame member 52. Strap 60 serves as a mechanism which: i) alone biases first frame member 42 and second frame member 52; or ii) which assists in biasing first frame member 42 and second frame member 52 in addition to another biasing mechanism. Strap 60 is formed from an elastic material (e.g., silicone, an elastic textile material, etc.) such that strap 60 acts generally as a spring. As shown schematically in FIG. 3, strap 60 may include one or more adjustment mechanisms 64 (e.g., hook and loop or any other suitable arrangement(s)) which allow for selective adjustment of the length L of strap 60 and thus the distance between first end 60a and second end 60b of strap 60.

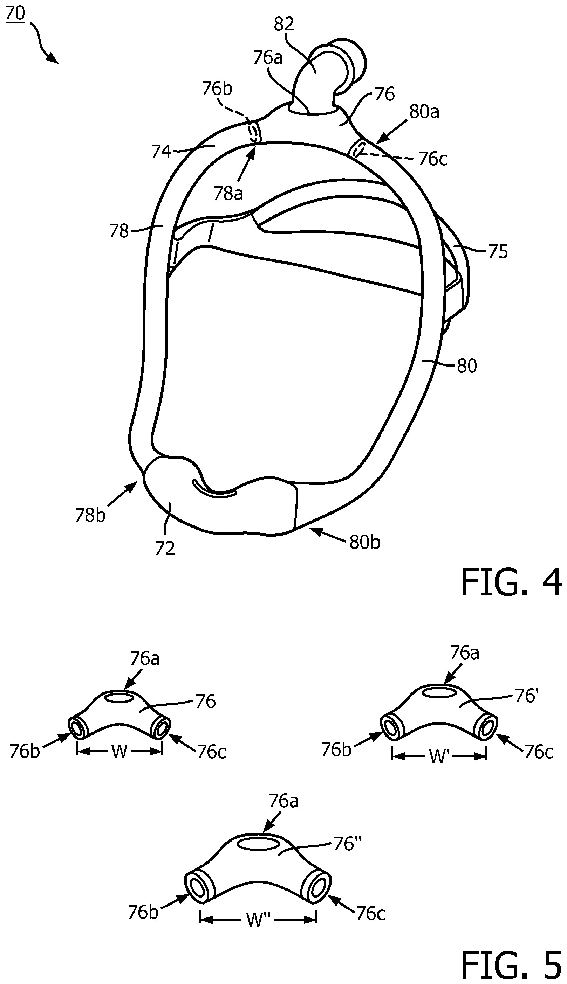

An example embodiment of another patient interface device, not according to the present invention, is shown in FIG. 4. Patient interface device 70 includes a patient sealing assembly 72 coupled to an example adjustable frame 74, not according to the present invention. In the example embodiment illustrated in FIG. 2, patient sealing assembly 72 is a nasal pillow, however, it is to be appreciated that other types of patient sealing assemblies, such as, without limitation, a nasal/oral mask, a nasal cushion, or any other suitable arrangements which facilitate the delivery of the flow of breathing gas to the airway of a patient may be substituted for patient sealing assembly 72. Patient interface device may also include a suitable headgear 75 for assisting in securing patient interface device 70 to the head of a patient.

Continuing to refer to FIG. 4, frame 74 includes a central hub member 76, a first frame member 78 and a second frame member 80. Central hub member 76 is formed as a generally hollow member and includes a central inlet 76a shown with an example elbow 82 coupled thereto, a first outlet 76b (shown in hidden line) and a second outlet 76c (shown in hidden line). First outlet 76a and second outlet 76b are disposed generally at opposite ends of central hub member 76 while central inlet 76a is disposed generally in a mid-portion of central hub member 76 between first outlet 76b and second outlet 76c. In use, elbow 82 is coupled to a suitable conduit (such as conduit 34 of FIG. 2) for receiving a flow of treatment gas therefrom. The flow of treatment gas is then communicated to the interior of central hub member via central inlet 76a and exits therefrom via either of first outlet 76b or second outlet 76c.

First frame member 78 includes a first end 78a, which is selectively coupled to first outlet 76b of central hub member 76, and an opposite second end 78b which is coupled to sealing assembly 72. First frame member 78 includes a flow passage (not numbered) defined therein which extends between first end 78a and second end 78b which is structured to convey a flow of treatment gas (such as provided by pressure/flow generator 32 of FIG. 2) received from central hub member 76 through first frame member 78. Second frame member 80 is of similar arrangement as first frame member 78. Accordingly, second frame member 80 has a first end 80a selectively coupled to second outlet 76b of central hub member 76, and an opposite second end 80b coupled to sealing assembly 72. Second frame member 80 also includes a passage (not numbered) defined therein which extends between first end 80a and second end 80b which is structured to convey a flow of treatment gas (such as provided by pressure/flow generator 32 of FIG. 2) received from central hub member 76 through second frame member 80.

Frame 74 may be selectively sized to a particular patient by replacing central hub member 76 (i.e., by selectively uncoupling from first frame member 78 and second frame member 80) with another central hub member of different size. FIG. 5 shows examples of three central hub members 76, 76' and 76" of different width W, W' and W" which may be employed in frame 74 of patient interface device 70 in sizing for a particular patient. Such variety of different sized hub members 76, 76' and 76" may be provided in a kit with patient interface device 70 or as a fitting kit provided separately from patient interface device 70. Although only three central hub members of differing width are shown, it is to be appreciated that one or both of the quantity and varied dimension of dimensions of central hub members may be varied.

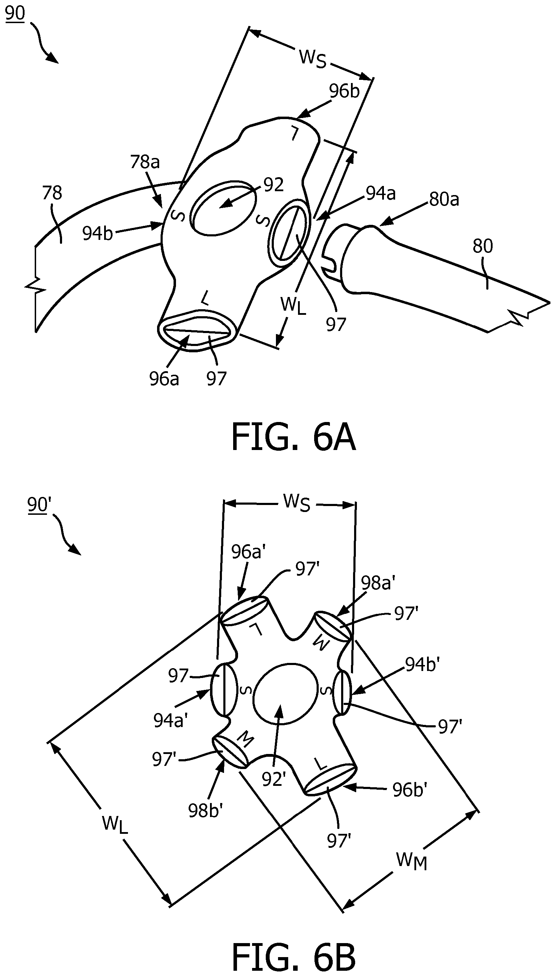

FIGS. 6A and 6B show examples of further central hub members 90 and 90' that may be employed to provide for selective sizing of patient interface device 70 of FIG. 4 to the head of a patient. Like central hub member 76, previously discussed, each of central hub members 90 and 90' is formed as a generally hollow member including a central a central inlet 92, 92', which is structured to be coupled to a conduit (e.g., via an elbow connector, not shown) providing a supply of a treatment gas, and a pair of opposing outlets 94a and 94b disposed apart a first distance W_{S} which are each structured to be selectively coupled to one of first end 78a of first frame member 78 and first end 80a of second frame member 80 of patient interface device 70 of FIG. 4. Unlike central hub member 76, central hub member 90 further includes an additional pair of opposing outlets 96a and 96b disposed apart a second distance W_{L}, greater than first distance Ws, which are each structured to be selectively coupled to one of first end 78a of first frame member 78 and first end 80a of second frame member of patient interface device 70 of FIG. 4. From such arrangement central hub member 90 provides for two different sizing choices in a single element dependent on which pair of outlets frame members 78 and 80 are coupled. In order to prevent treatment gas from escaping the unused pair of outlets, each of outlets 94a, 94b, 96a and 96b include a valve mechanism 97 (e.g., without limitation, a flapper valve) which is disposed on a closed position, thus preventing treatment gas from passing therethrough, when the associated outlet is not coupled to one of frame members 78 or 80. Central hub member 90' of similar to central hub member 90 except central hub member 90' includes yet a further pair of opposing outlets 98a' and 98b' disposed apart a third distance W_{M}, which is greater than Ws and less than W_{L}. Hence, central hub member provides for three different sizing possibilities dependent on the pair of outlets which are utilized.

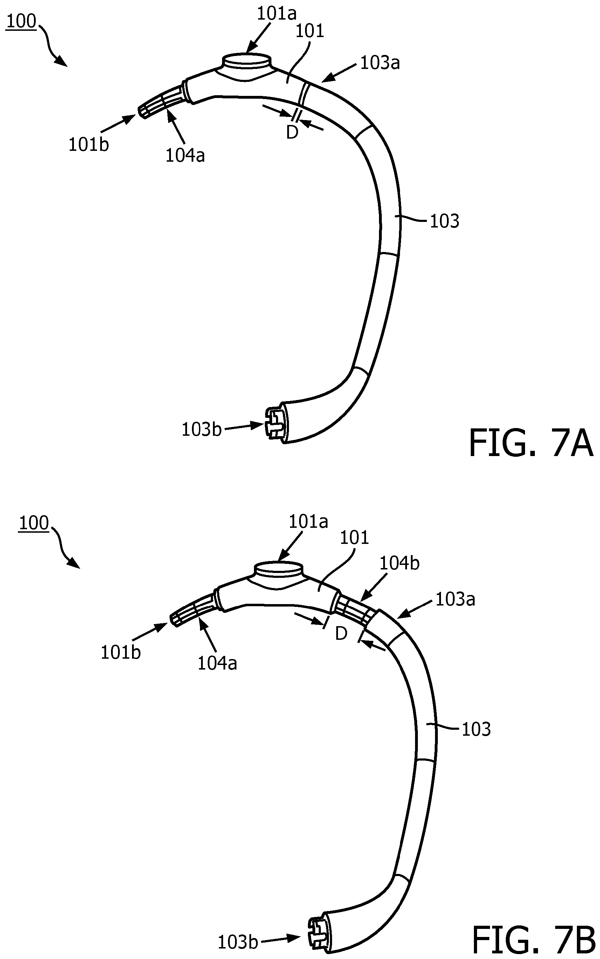

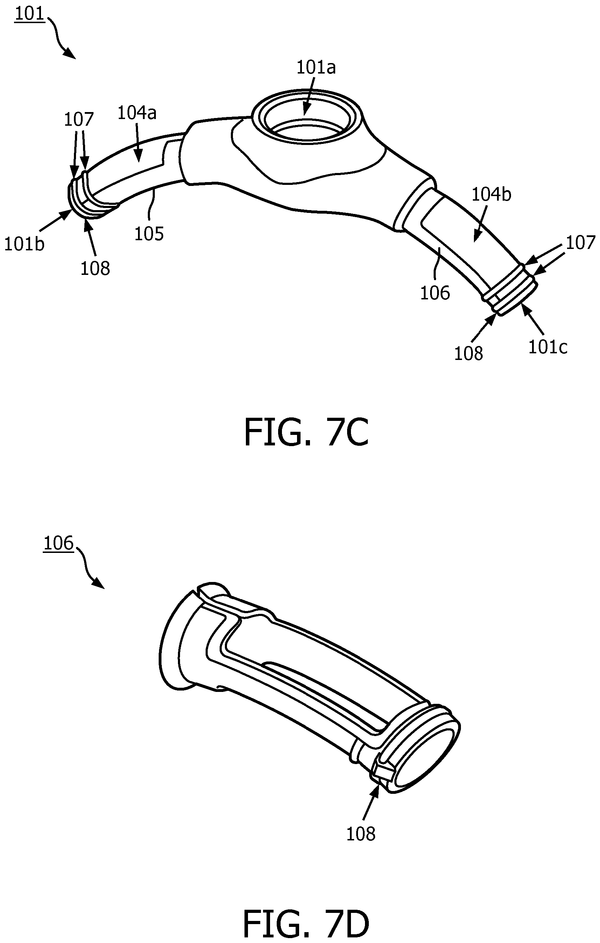

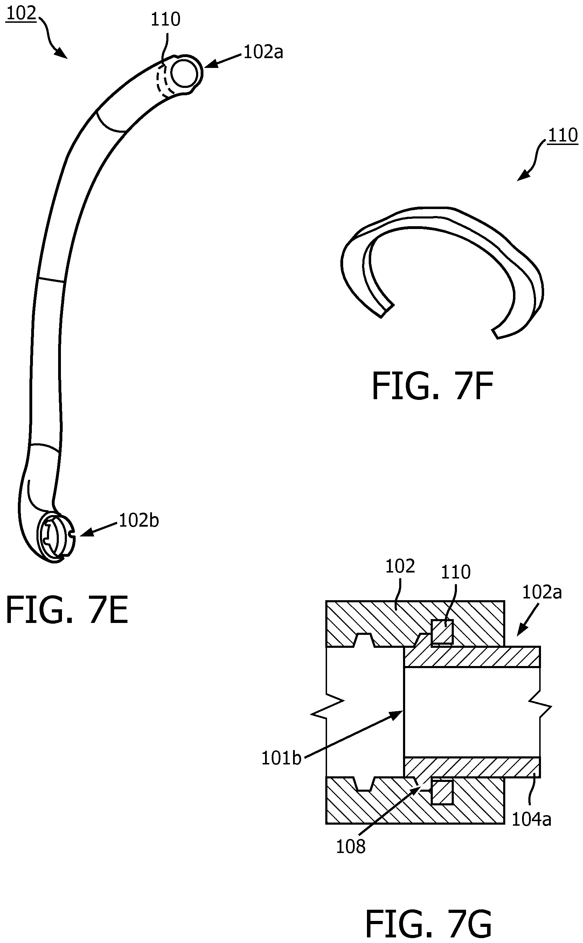

Selected portions of another example adjustable frame 100 which may be employed in a patient interface similar to patient interface device 36 of FIG. 2 are illustrated in FIGS. 7A and 7B. More particularly, frame 100 includes a generally hollow central hub member 101, which is also shown alone in FIG. 7C, and a pair of generally hollow arm members 102, 103 (only a left side, relative to the patient, arm member 103 is shown in the partial assembly of FIGS. 7A and 7B and a right side, relative to the patient, arm member 102 is shown alone in FIG. 7E). Referring to FIG. 7C, central hub member 101 includes an inlet 101a and a pair of opposing outlets 101b and 101c. Inlet 101a is structured to be coupled to a conduit (e.g., via an elbow connector) for receiving a flow of treatment gas. Each of outlets 101b and 101c are disposed at the end of reduced portions 104a and 104b of central hub member 101 which extend generally outward from inlet 101. Each reduced portion 104a, 104b is structured to be coupled to a corresponding first end 102a, 103a of a respective arm member 102, 103 such that treatment gas received at inlet 101a of central hub member 101 is communicated to each arm member 102, 103 and communicated therethrough to a second end 102b, 103b thereof and then to a sealing assembly (not shown), such as previously discussed, which may be coupled to second end 102b, 103b.

As demonstrated in the transition from FIG. 7A to FIG. 7B, the coupling between each reduced portion 104a and 104b of central hub member 101 and each arm member 102 and 103 is a generally telescoping-type slidable coupling which provides for a distance D between portions of central hub member 101 and each frame member 102, 103 to be selectively varied, thus generally providing for the size of frame 100 to be selectively varied. Tactile interlocks (not numbered) of any suitable arrangement may be provided between each reduced portion 104a, 104b and one or both of central hub member 101 and each arm member 102, 103 which provide for selective locking of each arm member 102, 103 and central hub member 104 in predetermined particular locations corresponding to predetermined sizes. Although shown as extensions of central hub member 101 in the example embodiment described in conjunction with FIGS. 7A-7G, it is to be appreciated that reduced portions 104a and 104b may be alternatively be formed as extensions of arm members 102 which slidingly engage into correspondingly sized outlets of a central hub member or as independent elements separate from each of a central hub member and arm members.

As shown in FIGS. 7C and 7D, each of reduced portions 104a and 104b may include a directional stiffener 105, 106 which is structured to generally increase stiffness (i.e., rigidity) of each reduced portion in one or more predetermined directions, while generally not increasing stiffness in one or more predetermined directions. Each stiffener 105, 106 may be formed: i) integrally with each reduced portion 104a, 104b; or ii) as a separate element which is then coupled (via mechanical or chemical means) to each reduced portion 104a, 104b. In the example illustrated in FIGS. 7C and 7D, stiffener 106 (and 105) is formed from a plastic of greater stiffness than reduced portion 104b which is forcibly slid onto reduced portion 104b. Each of reduced portions 104a and 104b and/or stiffeners 105 and 106 may include outward extending sealing bands 107 which extend around at a least a portion of the periphery of each reduced portion 104a, 104b and which are sized and structured to sealingly engage with an inside surface (not numbered) of a respective arm member 102, 103 in a manner which prevents gas leakage at the junctions between central hub member 101 and arm members 102 and 103.

As also shown in FIGS. 7C and 7D, each of reduced portions 104a and 104b and/or stiffeners 105 and 106 may include one or more outward extending locking tabs 108 which are each structured to engage a portion of each arm member 102, 103 in a manner which generally prohibits each arm member 102, 103 from being inadvertently slid completely off of the corresponding reduced portion 104a, 104b. In the example illustrated in FIGS. 7E-7G, each arm member 102, 103 includes a C-shaped rigid clip 110 which, as shown in FIG. 7E, is overmolded to arm member 102 (and similarly to arm member 103). As shown in the simplified sectional view of FIG. 7G, rigid clip 110 is positioned within arm member 102 so as to be engaged by locking tab 108 of reduced portion 104a in a manner which prohibits arm member 102 from disengaging with reduced portion 104a.

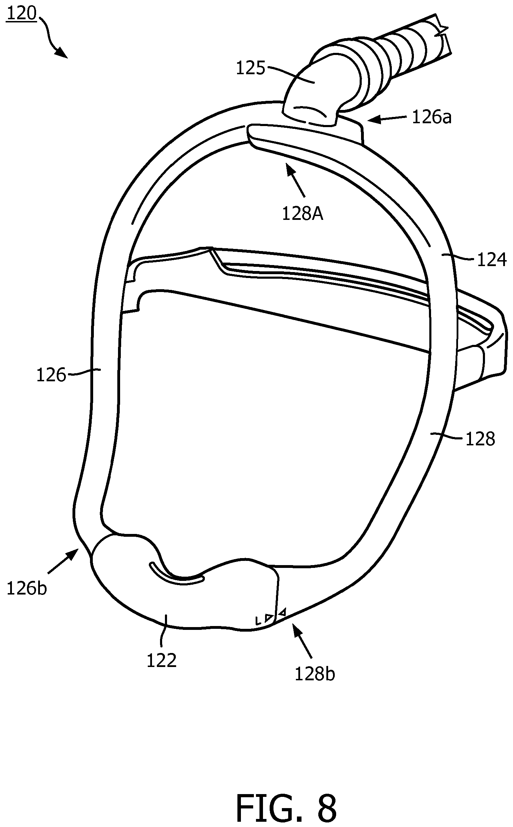

Another example patient interface 120, not according to the present invention, is shown in FIG. 8. Patient interface device 120 includes a patient sealing assembly 122 coupled to an example adjustable frame 124, not according to the present invention. In the example embodiment illustrated in FIG. 8, patient sealing assembly 122 is a nasal pillow, however, it is to be appreciated that other types of patient sealing assemblies, such as, without limitation, a nasal/oral mask, a nasal cushion, or any other suitable arrangements which facilitate the delivery of the flow of breathing gas to the airway of a patient may be substituted for patient sealing assembly 122.

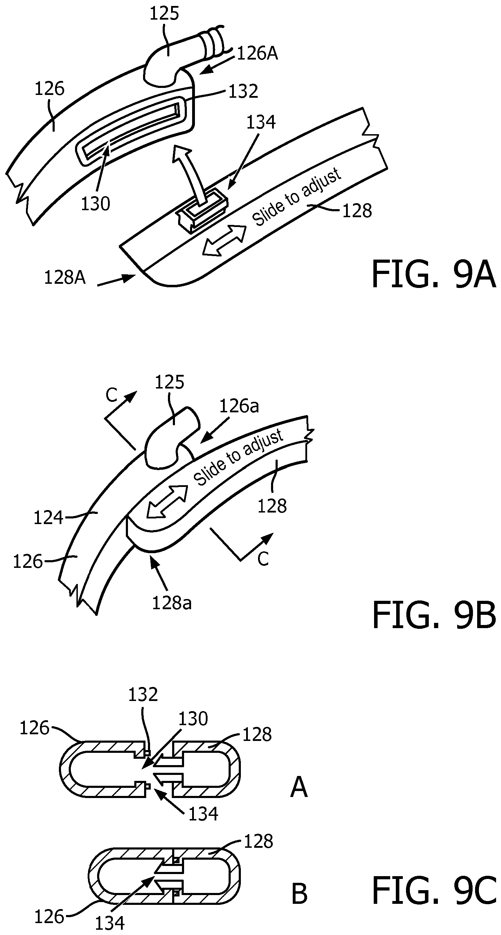

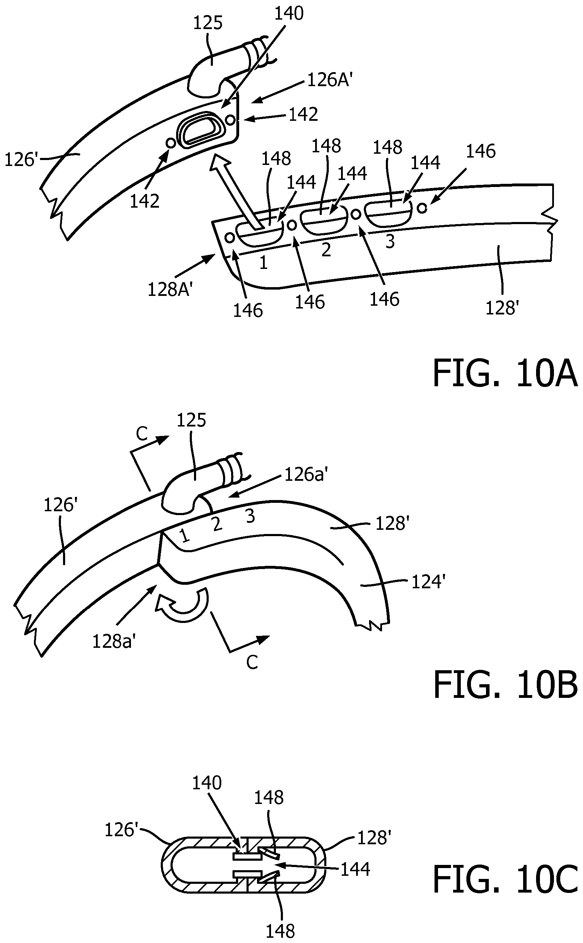

Continuing to refer to FIG. 8, frame 124 includes a first frame member 126 having a first end 126a which is structured to be coupled to a conduit (e.g., delivery conduit 34 of FIG. 2), such as via a suitable connector 125, supplying the flow of treatment gas and an opposite second end 126b coupled to sealing assembly 122. First frame member 126 includes a flow passage (not numbered) defined therein which extends between first end 126a and second end 126b which is structured to convey a flow of treatment gas (such as provided by pressure/flow generator 32 of FIG. 2) through first frame member 126. Frame 124 further includes a second frame member 128 of similar arrangement as first frame member 126. Accordingly, second frame member 128 has a first end 128a structured to be coupled to first end 126a od first frame member 126 and an opposite second end 128b coupled to sealing assembly 122. Second frame member 128 also includes a passage (not numbered) defined therein which extends between first end 128a and second end 128b which is structured to convey a flow of treatment gas (such as provided by pressure/flow generator 32 of FIG. 2) through second frame member 128. First frame member 126 and second frame member 128 are coupled together at or near first ends 126a and 128a thereof such that first ends 126A and 128A overlap. Example arrangements of such coupling between first ends 126A and 128A are shown respectively in FIGS. 9A-9C and 10A-10C.

Referring first to FIGS. 9A-9C, an example arrangement in which first ends 126A and 128A are adjustably coupled is shown. More particularly, first frame member 126 includes an elongated slot 130 defined therein at or about first end 126A. In the example embodiment illustrated in FIGS. 9A-9C, frame member 126 further includes a seal member 132 which encircles slot 130. Second frame member 128 includes a hollow protrusion 134 which extends from second frame member at or about first end 128A thereof. Hollow protrusion 134 is sized and configured to be slidingly engaged with slot 130 in a snap-fit manner such that first frame member and second frame member are coupled at or about first end 126A and first end 128A in a manner such that first frame member 126 and second frame member can slide with respect to each other, while remaining coupled. Furthermore, first frame member 126 and second frame member 128 are sealingly engaged (e.g., by seal member 132) when hollow protrusion 134 is positioned in any position within slot 130 such that a flow of a treatment gas provided to first frame member 126 (e.g., via connector 125) passes partly into second frame member 128 (and partly along first frame member 126) via hollow protrusion 134, and then further along second frame member 128 to second end 128b thereof. Hence, it is to be appreciated that such arrangement generally provides for the size of frame 124 to be selectively varied.

Referring now to FIGS. 10A-10C, another example arrangement in which first ends 126A and 128A of patient interface device 120 of FIG. 8, denoted as 126A' and 128A' in FIGS. 10A-10C, are adjustably coupled is shown. More particularly, in such example embodiment first frame member 126' includes a hollow protrusion 140 which extends from first frame member 126' at or about first end 126A' thereof and a number of first magnetic elements 142 (two are shown) coupled to first frame member 126' generally at or about hollow protrusion 140. Second frame member 128' includes a plurality of inlet openings 144 (three are shown) disposed at or about first end 128A' thereof which are each sized and configured to be engaged by hollow protrusion 140, such that hollow protrusion 140 can be selectively disposed in any one opening of inlet openings 144. Second frame member 128' further includes a number of second magnetic elements 146 which are positioned and structured to magnetically interact in an attractive manner with one or more of the number of first magnetic elements 142 of first frame member 126' when hollow protrusion 140 is disposed any one opening of inlet openings 144 such that first end 126a' of first frame member 126' and first end 128a' of second frame member 128' are coupled together. When hollow protrusion 140 is disposed in any one of inlet openings 144, a flow of a treatment gas provided to first frame member 126' (e.g., via connector 125) passes partly into second frame member 128' (and partly along first frame member 126') via hollow protrusion 140, and then further along second frame member 128' to an opposite second end (not numbered) thereof. Each of inlet openings 144 is provided with a valve mechanism 148 (e.g., without limitation, a number of flapper valves) in order to prevent the escape of treatment gas through one or more inlet openings 144 in which hollow protrusion 140 is not engaged. Hence, it is to be appreciated that the arrangement illustrated in FIGS. 10A-10C generally provides for the size of frame 124' to be selectively varied in an incremental manner (as determined by the quantity of inlet openings 144).

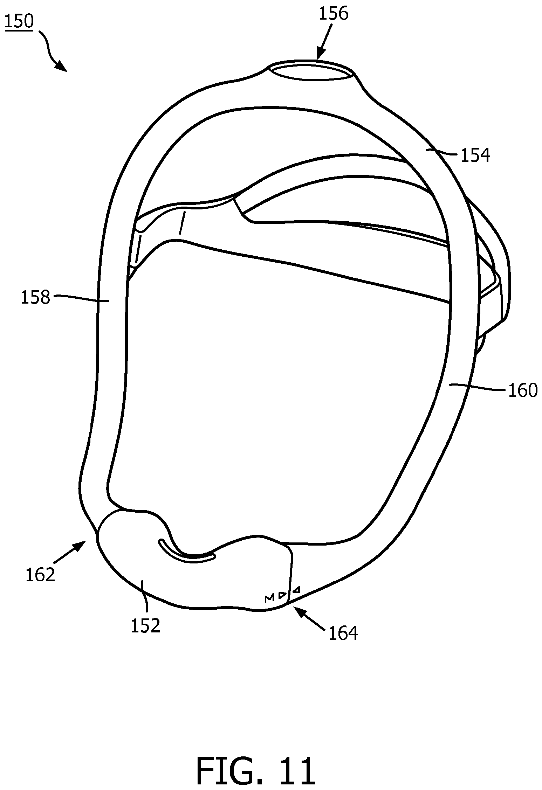

Yet another example patient interface 150, not according to the present invention, is shown in FIG. 11. Patient interface device 150 includes a patient sealing assembly 152 coupled to an example adjustable frame 154, not according to the present invention. In the example embodiment illustrated in FIG. 11, patient sealing assembly 152 is a nasal pillow, however, it is to be appreciated that other types of patient sealing assemblies, such as, without limitation, a nasal/oral mask, a nasal cushion, or any other suitable arrangements which facilitate the delivery of the flow of breathing gas to the airway of a patient may be substituted for patient sealing assembly 152.

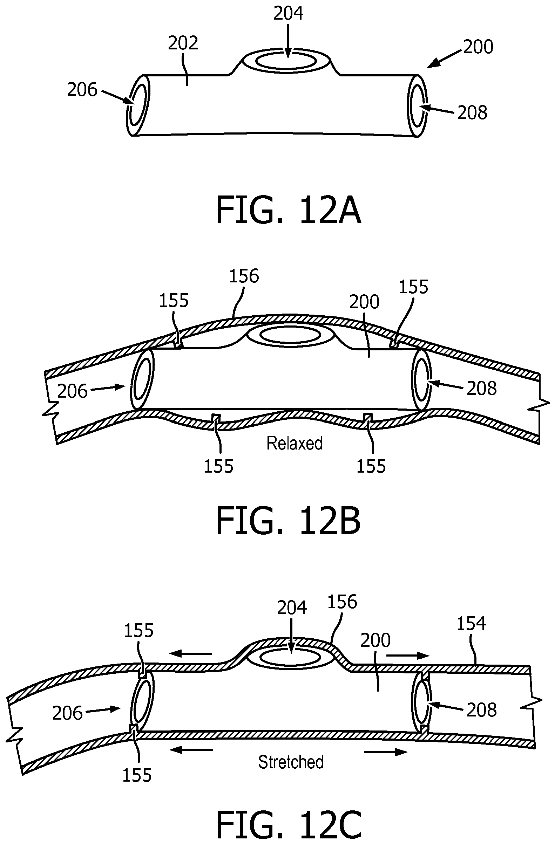

Continuing to refer to FIG. 11, frame 154 is formed from a flexible material (e.g., silicone) as a single element having a central opening 156 and a pair of arms 158, 160 which extend generally therefrom and terminate at respective ends 162, 164 which are coupled to sealing assembly 152. Frame 154 is formed as a generally hollow tubular member such that a flow of treatment gas provided to central opening 154 is conveyed to each of ends 162, 164 to sealing assembly 152 and finally to an airway of the patient engaged with sealing assembly 152. Central opening 156 is defined within a portion of frame 154 that is generally more flexible than the remainder of frame 154. Such extra flexibility may be accomplished via a thinner region, a region having a lower durometer, or any other suitable means. Such flexibility is utilized to house a generally hollow hub member, such as any of hub members 200, 300 or 400 of FIGS. 12A, 13A and 14A which provide for adjusting frame 154 in fitting patient interface device 150 to the head of a patient. In example embodiments, hub members 200, 300, 400 have been formed from a semi-rigid, yet flexible material, although other materials may be employed.

Referring to FIGS. 200, 300, and 400, each of hub members 200, 300 and 400 include: a main body 202, 302, 402, having a central inlet 204, 304, 404 which is structured to be coupled to a conduit (e.g., conduit 34 of FIG. 2) providing a flow of a treatment gas; a first outlet 206, 306, 406 which is positioned to supply first arm 158 of patient interface device 158 with a portion of a supply of treatment gas received at inlet 204, 304, 404; and a second outlet 208, 308, 408 which is positioned to supply second arm 160 of patient interface device 158 with a portion of a supply of treatment gas received at inlet 204, 304, 404.

Referring to FIG. 12B, frame 154 of patient interface device 150 is shown in a "relaxed" first positioning about hub 200 such that central inlet 204 of hub 200 is accessible via central opening 156 of frame 154. In such example embodiment frame 154 includes a number (four are shown) of inward extending protrusions 155 which are positioned on the interior of frame 154. Referring now to FIG.12C, frame 154 of patient interface device 150 is shown in a "stretched" second positioning about hub 200 such that central inlet 204 of hub 200 is accessible via central opening 156 of frame 154. In such example positioning, frame 154 has been stretched (i.e., elongated a predetermined amount) such that inward extending protrusions thereof are engaged with hub 200 about each of outlets 206 and 208. It is thus to be readily appreciated that such hub and deformable frame arrangement thus provides for two different sizings of frame 154 to be provided without changing any parts.

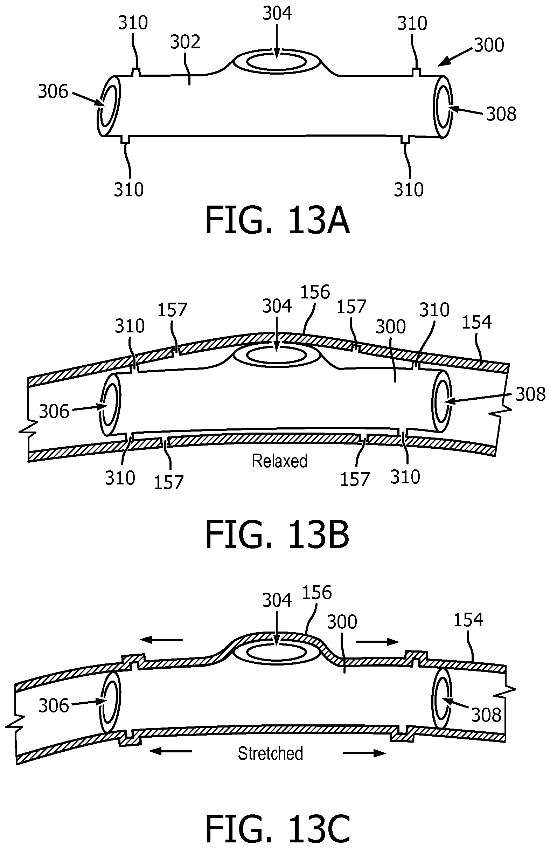

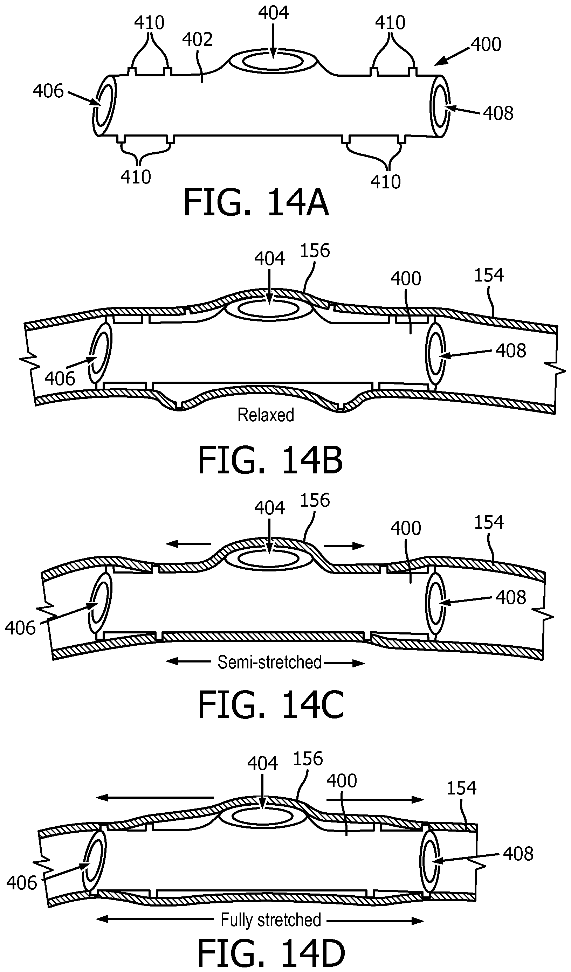

FIGS. 13A-13C and 14A-14C show hub/frame arrangements which operate in a similar manner as that of FIGS. 12A-12C. Referring to FIG. 13B, frame 154 of patient interface device 150 is shown in a "relaxed" first positioning about hub 300 such that central inlet 304 of hub 300 is accessible via central opening 156 of frame 154. In such example embodiment frame 154 includes a number (four are shown) of notches 157 defined in the interior surface of frame 154 which are engageable by a number (four are shown) of protrusions 310 which extend outward from main body 302 of hub 300. Referring now to FIG.13C, frame 154 of patient interface device 150 is shown in a "stretched" second positioning about hub 300 such that central inlet 304 of hub 300 is accessible via central opening 156 of frame 154. In such example positioning, frame 154 has been stretched (i.e., elongated a predetermined amount) such that notches 157 thereof are engaged by protrusions 310 of hub 300.

FIGS. 14A-14C show a hub/frame arrangement similar to that of FIGS. 13A-13C but which offers three different sizing positions. Referring to FIG. 14B, frame 154 of patient interface device 150 is shown in a "relaxed" first positioning about hub 400 such that central inlet 404 of hub 400 is accessible via central opening 156 of frame 154. In such example embodiment frame 154 includes a number (four are shown) of notches 157 defined in the interior surface of frame 154 which are engageable by a number (eight are shown) of protrusions 410 which extend outward from main body 402 of hub 400. Protrusions 410 are generally positioning in two groupings, an "inner" grouping and an "outer" grouping. Referring now to FIG.14C, frame 154 of patient interface device 150 is shown in a "semi-stretched" second positioning about hub 400 such that central inlet 404 of hub 400 is accessible via central opening 156 of frame 154. In such example positioning, frame 154 has been stretched a relatively small amount (i.e., elongated a predetermined first amount) such that notches 157 thereof are engaged by "inner" protrusions 410 of hub 400. Referring finally to FIG. 14D, frame 154 of patient interface device 150 is shown in a "frilly stretched" third positioning about hub 400 such that central inlet 404 of hub 400 is accessible via central opening 156 of frame 154. In such example positioning, frame 154 has been stretched a larger amount (i.e., elongated a predetermined second amount) such that notches 157 thereof are engaged by "outer" protrusions 410 of hub 400.

In the claims, any reference signs placed between parentheses shall not be construed as limiting the claim. The word "comprising" or "including" does not exclude the presence of elements or steps other than those listed in a claim. In a device claim enumerating several means, several of these means may be embodied by one and the same item of hardware. The word "a" or "an" preceding an element does not exclude the presence of a plurality of such elements. In any device claim enumerating several means, several of these means may be embodied by one and the same item of hardware. The mere fact that certain elements are recited in mutually different dependent claims does not indicate that these elements cannot be used in combination.

## Claims

1. A frame (40) for use in an interface device (36) having a sealing assembly (38) for delivering a flow of treatment gas to the airway of a patient, the frame comprising:
a first frame member (42) having a first end (42a) structured to be coupled to a conduit (34) supplying the flow of treatment gas and an opposite second end (42b) structured to be coupled to the sealing assembly, the first frame member having a passage defined therein which extends between the first end and the second end thereof;
a second frame member (52) having a first end (52a) structured to be coupled to the conduit supplying the flow of treatment gas and an opposite second (52b) end structured to be coupled to the sealing assembly, the second frame member having a passage defined therein which extends between the first end and the second end thereof; and
a strap (60) coupled between the first frame member and the second frame member, wherein the strap (60) comprises a first end (60a) and an opposite second end (60b), wherein the first end of the strap is coupled to the first frame member at a location between the first end and the second end of the first frame member, and wherein the second end of the strap is coupled to the second frame member at a location between the first end and the second end of the second frame member;
wherein the first frame member is disposed on a first side of a reference plane (P) which passes through the sealing assembly and the second frame member is disposed on a second side of the reference plane opposite the first side, and
**characterized in that** the strap serves to bias at least one of the first frame member and the second frame member toward the other of the first frame member and the second frame member, and the strap is formed from an elastic material such that the strap acts like a spring.

2. The frame (40) of claim 1, wherein the first end (42a) of the first frame member (42) and the first end (52a) of the second frame member (52) are merged together in a single inlet conduit (56) which is structured to be coupled to the conduit (34) supplying the flow of treatment gas.

3. An interface device (36) for use in delivering a flow of treatment gas to the airway of a user of the device, the device comprising:
(a) a sealing assembly (38) having a compartment defined therein, a first opening having a perimeter structured to sealingly engage about at least one airway of the user of the device, a second opening, and a third opening; and
(b) a frame (40) according to claim 1, the frame being structured to couple the sealing assembly to the head of the user, wherein the second end (42b) of the first frame member (42) is coupled to the second opening of the sealing assembly and the second end (52b) of the second frame member (52) is coupled to the third opening of the sealing assembly.

4. The interface device (36) of claim 3, wherein the first end (42a) of the first frame member (42) and the first end (52a) of the second frame member (52) are merged together in a single inlet conduit (56) which is structured to be coupled to the conduit (34) supplying the flow of treatment gas.

5. A system (30) for use in delivering a flow of a breathing gas to the airway of a patient, the system comprising:
(a) a pressure generating device (32);
(b) a conduit (34) having a first end coupled to the pressure generating device and an opposite second end; and
(c) an interface device (36) comprising:
(1) a sealing assembly (38) having a compartment defined therein, a first opening having a perimeter structured to sealingly engage about the airway of the patient, a second opening, and a third opening; and
(2) a frame (40) according to claim 1, the frame being structured to couple the sealing assembly to the head of the patient, wherein the first end (42a) of the first frame member (42) is coupled to the second end of the conduit and the first end (52a) of the second frame member (52) is coupled to the second end of the conduit, and wherein the second end (42b) of the first frame member is coupled to the second opening of the sealing assembly and the second end (52b) of the second frame member is coupled to the third opening of the sealing assembly.

6. A system (30) according to claim 5, wherein the first end (42a) of the first frame member (42) and the first end (52a) of the second frame member (52) are merged together in a single inlet conduit (56) which is coupled to the conduit (34) supplying the flow of treatment gas.

## Patentansprüche

1. Rahmen (40) zur Verwendung in einer Schnittstellenvorrichtung (36), die eine Dichtungsanordnung (38) zur Abgabe eines Behandlungsgasstroms an die Atemwege eines Patienten aufweist, wobei der Rahmen umfasst:
ein erstes Rahmenelement (42), das ein erstes Ende (42a) aufweist, das strukturiert ist, um mit einer Leitung (34) zum Zuführen des Behandlungsgasstroms gekoppelt zu werden, und ein gegenüberliegendes zweites Ende (42b), das strukturiert ist, um mit der Dichtungsanordnung gekoppelt zu werden, wobei das erste Rahmenelement einen darin definierten Durchgang aufweist, der sich zwischen dem ersten Ende und dem zweiten Ende davon erstreckt;
ein zweites Rahmenelement (52), das ein erstes Ende (52a) aufweist, das strukturiert ist, um mit der Leitung zum Zuführen des Behandlungsgasstroms gekoppelt zu werden, und ein gegenüberliegendes zweites Ende (52b), das strukturiert ist, um mit der Dichtungsanordnung gekoppelt zu werden, wobei das zweite Rahmenelement einen darin definierten Durchgang aufweist, der sich zwischen dem ersten Ende und dem zweiten Ende davon erstreckt; und
einen Riemen (60), der zwischen dem ersten Rahmenelement und dem zweiten Rahmenelement gekoppelt ist, wobei der Riemen (60) ein erstes Ende (60a) und ein gegenüberliegendes zweites Ende (60b) umfasst, wobei das erste Ende des Riemens an einer Stelle zwischen dem ersten Ende und dem zweiten Ende des ersten Rahmenelements mit dem ersten Rahmenelement gekoppelt ist und wobei das zweite Ende des Riemens an einer Stelle zwischen dem ersten Ende und dem zweiten Ende des zweiten Rahmenelements mit dem zweiten Rahmenelement gekoppelt ist;
wobei das erste Rahmenelement auf einer ersten Seite einer Referenzebene (P) angeordnet ist, die durch die Dichtungsanordnung verläuft, und das zweite Rahmenelement auf einer zweiten Seite der Referenzebene gegenüber der ersten Seite angeordnet ist, und
**dadurch gekennzeichnet, dass** der Riemen dazu dient, mindestens eines von dem ersten Rahmenelement und dem zweiten Rahmenelement in Richtung des anderen von dem ersten Rahmenelement und dem zweiten Rahmenelement vorzuspannen, und der Riemen aus einem elastischen Material gebildet ist, sodass der Riemen wie eine Feder wirkt.

2. Rahmen (40) nach Anspruch 1, wobei das erste Ende (42a) des ersten Rahmenelements (42) und das erste Ende (52a) des zweiten Rahmenelements (52) in einer einzigen Einlassleitung (56) zusammengeführt sind, die strukturiert ist, um an die Leitung (34) gekoppelt zu werden, die den Behandlungsgasstrom zuführt.

3. Schnittstellenvorrichtung (36) zur Verwendung der Abgabe eines Behandlungsgasstroms an die Atemwege eines Benutzers der Vorrichtung, wobei die Vorrichtung umfasst:
(a) eine Dichtungsanordnung (38), die ein darin definiertes Fach, eine erste Öffnung, die einen Umfang aufweist, der strukturiert ist, um dichtend um mindestens einen Atemweg des Benutzers der Vorrichtung herum einzugreifen, eine zweite Öffnung und eine dritte Öffnung aufweist; und
(b) einen Rahmen (40) nach Anspruch 1, wobei der Rahmen strukturiert ist, um die Dichtungsanordnung mit dem Kopf des Benutzers zu koppeln, wobei das zweite Ende (42b) des ersten Rahmenelements (42) mit der zweiten Öffnung der Dichtungsanordnung gekoppelt ist und das zweite Ende (52b) des zweiten Rahmenelements (52) mit der dritten Öffnung der Dichtungsanordnung gekoppelt ist.

4. Schnittstellenvorrichtung (36) nach Anspruch 3, wobei das erste Ende (42a) des ersten Rahmenelements (42) und das erste Ende (52a) des zweiten Rahmenelements (52) in einer einzigen Einlassleitung (56) zusammengeführt sind, die strukturiert ist, um mit der Leitung (34) gekoppelt zu werden, die den Behandlungsgasstrom zuführt.

5. System (30) zur Verwendung bei der Abgabe eines Stroms eines Atemgases an die Atemwege eines Patienten, wobei das System umfasst:
(a) eine Druckerzeugungsvorrichtung (32);
(b) eine Leitung (34), die ein erstes Ende, das mit der Druckerzeugungsvorrichtung gekoppelt ist, und ein gegenüberliegendes zweites Ende aufweist; und
(c) eine Schnittstellenvorrichtung (36), umfassend:
(1) eine Dichtungsanordnung (38), die ein darin definiertes Fach, eine erste Öffnung, die einen Umfang aufweist, der strukturiert ist, um dichtend um mindestens einen Atemweg des Patienten herum einzugreifen, eine zweite Öffnung und eine dritte Öffnung aufweist; und
(2) einen Rahmen (40) nach Anspruch 1, wobei der Rahmen strukturiert ist, um die Dichtungsanordnung mit dem Kopf des Benutzers zu koppeln, wobei das erste Ende (42a) des ersten Rahmenelements (42) an das zweite Ende der Leitung gekoppelt ist und das erste Ende (52a) des zweiten Rahmenelements (52) an das zweite Ende der Leitung gekoppelt ist, und wobei das zweite Ende (42b) des ersten Rahmenelements mit der zweiten Öffnung der Dichtungsanordnung gekoppelt ist, und das zweite Ende (52b) des zweiten Rahmenelements mit der dritten Öffnung der Dichtungsanordnung gekoppelt ist.

6. System (30) nach Anspruch 5, wobei das erste Ende (42a) des ersten Rahmenelements (42) und das erste Ende (52a) des zweiten Rahmenelements (52) in einer einzigen Einlassleitung (56) zusammengeführt sind, die mit der Leitung (34) gekoppelt ist, die den Behandlungsgasstrom zuführt.

## Revendications

1. Cadre (40) destiné à être utilisé dans un dispositif d'interface (36) présentant un ensemble d'étanchéité (38) destiné à distribuer un flux de gaz de traitement aux voies respiratoires d'un patient, le cadre comprenant :
un premier élément de cadre (42) présentant une première extrémité (42a) structurée pour être couplée à un conduit (34) acheminant le flux de gaz de traitement et une seconde extrémité opposée (42b) structurée pour être couplée à l'ensemble d'étanchéité, le premier élément de cadre présentant un passage défini en son sein qui s'étend entre la première extrémité et la seconde extrémité de celui-ci ;
un second élément de cadre (52) présentant une première extrémité (52a) structurée pour être couplée au conduit acheminant le flux de gaz de traitement et une seconde extrémité opposée (52b) structurée pour être couplée à l'ensemble d'étanchéité, le second élément de cadre présentant un passage défini en son sein qui s'étend entre la première extrémité et la seconde extrémité de celui-ci ; et
une sangle (60) couplée entre le premier élément de cadre et le second élément de cadre, dans lequel la sangle (60) comprend une première extrémité (60a) et une seconde extrémité opposée (60b), dans lequel la première extrémité de la sangle est couplée au premier élément de cadre en un emplacement entre la première extrémité et la seconde extrémité du premier élément de cadre, et dans lequel la seconde extrémité de la sangle est couplée au second élément de cadre en un emplacement entre la première extrémité et la seconde extrémité du second élément de cadre ;
dans lequel le premier élément de cadre est disposé sur un premier côté d'un plan de référence (P) qui passe à travers l'ensemble d'étanchéité, et le second élément de cadre est disposé sur un second côté du plan de référence opposé au premier côté, et
**caractérisé en ce que** la sangle sert à solliciter au moins l'un du premier élément de cadre et du second élément de cadre vers l'autre du premier élément de cadre et du second élément de cadre, et la sangle est formée à partir d'un matériau élastique de telle sorte que la sangle agisse comme un ressort.

2. Cadre (40) selon la revendication 1, dans lequel la première extrémité (42a) du premier élément de cadre (42) et la première extrémité (52a) du second élément de cadre (52) sont fusionnées ensemble dans un conduit d'entrée unique (56) qui est structuré pour être couplé au conduit (34) acheminant le flux de gaz de traitement.

3. Dispositif d'interface (36) destiné à être utilisé pour distribuer un flux de gaz de traitement aux voies respiratoires d'un utilisateur du dispositif, le dispositif comprenant :
(a) un ensemble d'étanchéité (38) présentant un compartiment défini en son sein, une première ouverture présentant un périmètre structuré pour venir en prise étanche autour d'au moins une voie aérienne de l'utilisateur du dispositif, une deuxième ouverture et une troisième ouverture ; et
(b) un cadre (40) selon la revendication 1, le cadre étant structuré pour coupler l'ensemble d'étanchéité à la tête de l'utilisateur, dans lequel la seconde extrémité (42b) du premier élément de cadre (42) est couplée à la deuxième ouverture de l'ensemble d'étanchéité et la seconde extrémité (52b) du second élément de cadre (52) est couplée à la troisième ouverture de l'ensemble d'étanchéité.

4. Dispositif d'interface (36) selon la revendication 3, dans lequel la première extrémité (42a) du premier élément de cadre (42) et la première extrémité (52a) du second élément de cadre (52) sont fusionnées ensemble dans un conduit d'entrée unique (56) qui est structuré pour être couplé au conduit (34) acheminant le flux de gaz de traitement.

5. Système (30) destiné à être utilisé pour distribuer un flux de gaz respiratoire aux voies respiratoires d'un patient, le système comprenant :
(a) un dispositif générateur de pression (32) ;
(b) un conduit (34) présentant une première extrémité couplée au dispositif générateur de pression et une seconde extrémité opposée ; et
(c) un dispositif d'interface (36) comprenant :
(1) un ensemble d'étanchéité (38) présentant un compartiment défini en son sein, une première ouverture présentant un périmètre structuré pour venir en prise étanche autour des voies respiratoires du patient, une deuxième ouverture et une troisième ouverture ; et
(2) un cadre (40) selon la revendication 1, le cadre étant structuré pour coupler l'ensemble d'étanchéité à la tête du patient, dans lequel la première extrémité (42a) du premier élément de cadre (42) est couplée à la seconde extrémité du conduit et la première extrémité (52a) du second élément de cadre (52) est couplée à la seconde extrémité du conduit, et dans lequel la seconde extrémité (42b) du premier élément de cadre est couplée à la deuxième ouverture de l'ensemble d'étanchéité et la seconde extrémité (52b) du second élément de cadre est couplée à la troisième ouverture de l'ensemble d'étanchéité.

6. Système (30) selon la revendication 5, dans lequel la première extrémité (42a) du premier élément de cadre (42) et la première extrémité (52a) du second élément de cadre (52) sont fusionnées ensemble dans un conduit d'entrée unique (56) qui est couplé au conduit (34) acheminant le flux de gaz de traitement.
